# EUROPEAN PATENT APPLICATION

(11) **EP 1 584 914 A1**
(43) Date of publication of application: **12.10.2005**
(21) Application number: 04008531.8
(22) Date of filing: 08.04.2004
(51) Int. Cl.: G01N 21/21, C12Q 1/68, G01N 33/543

(54) **A method of detecting a hybrid, formed of at least two species, on a substrate using ellipsometry**

(71) Applicant: Sony Deutschland GmbH, 50829 Köln (DE)
(72) Inventor: Kügler, Ralf, 70327 Stuttgart (DE); Basquin, Claire, 70327 Stuttgart (DE)
(74) Representative: Appelt, Christian W.

(57) **Abstract**

The present invention relates to a label-free method of detecting a hybrid, such as a double stranded nucleic acid, a protein - DNA complex or an antibody - antigen-complex, formed by at least two species, such as two single stranded nucleic acids, a protein and a nucleic acid, or an antibody and an antigen, on a substrate over time.

## Description

The present invention relates to a label-free method of detecting a hybrid, such as a double stranded nucleic acid, a protein - DNA complex or an antibody - antigen-complex, formed by at least two species, such as two single stranded nucleic acids, a protein and a nucleic acid, or an antibody and an antigen, on a substrate over time.

The use of biomolecular species, such as nucleic acids which are immobilized on a substrate is becoming increasingly important in the fields of medical diagnostics and analysis, forensic analysis as well as the labeling and identification of objects. For example, in the field of medical diagnostics, DNA-chips are used which essentially comprise a surface to which a number of single stranded nucleic acid capture molecules are attached at defined places, whereupon this construction is exposed to a solution, for example a sample prepared from the body fluid of a patient. The sample is suspected of containing a nucleic acid that is indicative of an infection being present in said patient or indicative of said patient showing a specific genetic trait. In the preparation of the DNA-chip, the capture molecules have been selected such that they would hybridize to these nucleic acids suspected of being present in the sample prepared from the patient's body fluid. If such nucleic acid is, in fact, present in the sample, a hybridization event occurs, and the presence of the hybrid is detected.

Such a nucleic acid chip is for example described in European patent EP 0 373 203 which discloses a structured array carrying a set of oligonucleotides, wherein the different oligonucleotides occupy separate cells of the array and are capable of taking part in hybridization reactions. The array described in EP 0 373 203 may comprise one or several types of oligonucleotides, each type of oligonucleotide being defined by its unique sequence. To this array a sample is added containing a polynucleotide sequence or fragments thereof, which have been labeled, and hybridization is allowed to occur. The label disclosed in EP 0 373 203 is a radioactive nucleotide. The DNA-chip is used for the analysis of known point mutations, genomic fingerprinting, linkage analysis, characterization of mRNAs, mRNA populations and sequence determination.

EP 0 820 524 describes a method for detecting DNA-sequence variations using a nucleic acid chip similar to the one described in EP 0 373 203 wherein radioactive and/or fluorescent labels are used. The methods of detection disclosed therein comprise autoradiography and fluorescence detection.

Furthermore a considerable amount of literature exists which deals with the characterization of single stranded nucleic acids which have been bound to a substrate surface. Various techniques have been used to study such surface immobilized single stranded nucleic acids. These include surface plasmon fluorescence spectroscopy, neutron reflectivity measurements, and X-ray photoelectron spectroscopy (XPS). Neumann et al. (Adv. Funct. Mater. 2002, 12, No. 9, 575-586), describe a way of surface-plasmon fluorescence spectroscopy applied to nucleic acid hybridization events, wherein surface-attached oligonucleotide capture probes hybridize to complementary target strands from solution. Different modes of operation in such surface-plasmon field-enhanced fluorescence spectroscopy are described. Either the target or the probes may be labeled; intercalating dyes may be used; a fluorescence resonance energy transfer event (FRET) is initiated between a donor dye at the probe strand and an acceptor dye at the target sequence, or competitive binding between a single stranded binding protein (SSB) and a chromophor-labeled target strand is initiated. In any case, an external label is used for this technique, introducing potential artifacts into the system.

Levicky et al. (J. Am. Chem. Soc. 1998, 120, 9787-9792) use neutron reflectivity measurements to determine the concentration profiles of oligomeric DNA monolayers on gold in high salt concentrations. The measurements are logistically laborious and do not allow for a temporal resolution of the formation of nucleic acid hybrids.

Heme et al. (J. Am. Chem. Soc. 1997, 119, 8916-8920) use ³²P-radiolabeling to characterize hybridization events between immobilized single stranded DNA on gold and complementary DNA oligonucleotides, which where radio-labeled with ³²P. No time resolution of the hybridization event and/or the immobilization event on the surface can be achieved.

In summary, the techniques as disclosed in the prior art, either use labor intense methods which may involve the use of artificial labels, and none of these allow for a temporal resolution of hybridization events, occurring on a surface, irrespective of whether such formation of hybrid takes place between two single stranded nucleic acids, two proteins, a nucleic acid and a protein, an antibody and an antigen etc.

Accordingly it was an object of the present invention to provide a method that allows to follow the immobilization of a biomolecular species, such as a nucleic acid, on a surface over time as well as the hybridization between such an immobilized nucleic acid probe and a nucleic acid complementary thereto or between an immobilised nucleic acid and a protein etc. Furthermore, it was an object of the present invention to provide for such a detection method that does not require molecular labeling. Also it was an object to provide for a method which is not restricted to specific substrate materials.

Another object of the present invention was to provide for a method that allows to optimize the hybridization efficiency with respect to hybridization conditions.

Moreover, it was an object of the present invention to provide a method that allows the detection of mismatches between hybrid forming species, e.g. the detection of oligonucleotides mismatches.

All these objects are solved by a method of detecting a hybrid, formed of at least two species, on a substrate, comprising the steps:
a) providing a substrate and a first species,
b) attaching said first species to said substrate,
c) providing a second species, suspected of being able to interact with said first species so as to form a hybrid,
d) allowing formation of said hybrid, if any, out of said first species and said second species to occur, and
e) following said formation of said hybrid over time by means of ellipsometry.

In one embodiment, said hybrid, formed of at least two species, is a complex of said at least two species.

Preferably, said step b) occurs via adsorption or covalent linkage.

In one embodiment, the method according to the present invention comprises the optional step:
ba) following the attachment of said first species to said substrate over time by means of ellipsometry.

In a preferred embodiment any of steps a) - e) and any substeps occurs without any radioactive, fluorescent, other optical and/or enzymatic label being present.

In one embodiment, said formation of said hybrid is allowed to occur in solution, preferably aqueous solution.

In one embodiment, said formation of said hybrid is allowed to occur under the influence of an electric field, preferably an AC electric field.

Preferably, said formation of said hybrid is allowed to occur at a pH in the range of from 2 - 12, an ionic strength in the range of from 1 mM to 1 M, and/or a temperature in the range of from 10°C to 80°C.

In one embodiment, said formation of said hybrid is recorded as a signal-over-time-ellipsometry-data so as to allow a subsequent analysis of said signal-over-time-data.

In a preferred embodiment, said ellipsometry is performed using a data acquisition rate of > 0.2 Hz, an incident wavelength in the range of from 380 nm to 900 nm and an incident angle in the range of from 40° - 80°.

Preferably, said ellipsometry is selected from the group comprising ellipsometry and attenuated-total-reflection ellipsometry (ATR).

In one embodiment said substrate is selected from the group comprising metals, preferably gold, Si, and glass.

In one embodiment, said first species is attached to nanoparticles or nanorods of a material selected from the group comprising metals, preferably gold, silica, carbon and polymeric substances, wherein preferably, said first species are attached to said nanoparticles or said nanorods before step b) or step c).

In one embodiment, said at least two species are selected from the group comprising nucleic acids and proteins.

In one embodiment, said first species is an antibody and said second species is an antigen or vice versa.

In one embodiment, said first species is a nucleic acid or a protein or a protein-nucleic acid complex, and said second species is a nucleic acid or a protein or a protein-nucleic acid-complex.

Preferably, said first species is a nucleic acid and said second species is a nucleic acid, wherein, more preferably, said first species is a single-stranded nucleic acid and said second species is a single-stranded nucleic acid.

In one embodiment, said second single-stranded nucleic acid is suspected of being complementary to said first single-stranded nucleic acid over 80%, preferably over 90%, more preferably over 95% of the length of first said single-stranded nucleic acid.

In another embodiment, said second single-stranded nucleic acid is suspected of being complementary to said first single-stranded nucleic acid over the entire length over said first single-stranded nucleic acid.

Preferably, said first and/or said second single-stranded nucleic acids have a length of 10 - 100, preferably 10 - 80, more preferably 10 - 50, most preferably 10 - 30 nucleotides.

In one embodiment, said first and/or said second nucleic acids are selected from the group comprising DNA, RNA, PNA and other types of modifies nucleic acids.

The objects of the invention are also solved by the use of ellipsometry for following the formation of a hybrid, as defined above, from at least two species, as defined above, on a substrate, or the attachment of a species to a substrate, over time.

The inventors have surprisingly found that by using ellipsometry, a time resolved measurement of hybridization events or hybrid formation on substrate surfaces can be performed. The method has no artifact associated in that it excludes artificial labels present in the sample and it yields temporally resolved data that allow the determination of binding coefficients and affinity constants between the hybrid forming species, such as single stranded nucleic acids involved in the formation of a duplex as well as single stranded nucleic acids and proteins or antibody / antigen forming hybrids, respectively.

As used herein the term "ellipsometry" is meant to designate a detection method whereby electromagnetic radiation (UV-VIS, infrared, preferably UV-VIS) is irradiated on and reflected from a surface, as a result of which the two perpendicular components of the electromagnetic field, which in the incident radiation are parallel and perpendicular to the incident plane, have been changed with respect to their absolute amount as well as with respect to their phase relation.

Ellipsometry can detect thin film layer thicknesses and their changes in the Angstrom range. The method is not restricted to a special substrate material and can be used either in air or liquid environment. Ellipsometry, as used in the context of the present invention, is used to follow hybridization events between single stranded nucleic acids, antibody / antigen or nucleic acid / protein hybrid formation over time and/or the adsorption of a single stranded nucleic acid on a substrate surface over time. The adsorption can be described depending on its nature. Theoretical models are applied in order to determine the efficiency of the surface reaction.
An example model is the Langmuir type model for a limited number of equivalent binding sites on a surface which can be occupied by binding molecules in solution until the complete surface is saturated and all sites are blocked.

Likewise, hybridization events can be described by the Langmuir type model. In a nutshell, the Langmuir-type time dependency of a measured value can be described by the formula *I* (*t*) = *I*_{∞}(1 - exp - (*k*_{*on*} *c*_{*o*} + *k*_{*off*}) *t*), wherein I is the measured value, *I*_{∞} the equilibrium value of the completed reaction, *k*_{*on*} the association rate, *k*_{*off*} the dissociation rate and *c*_{*o*} the concentration of the reaction partner in solution, respectively.

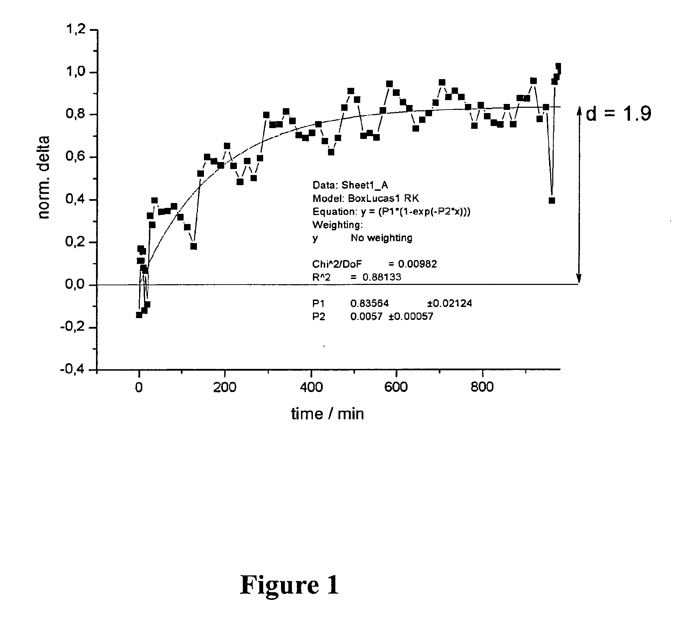

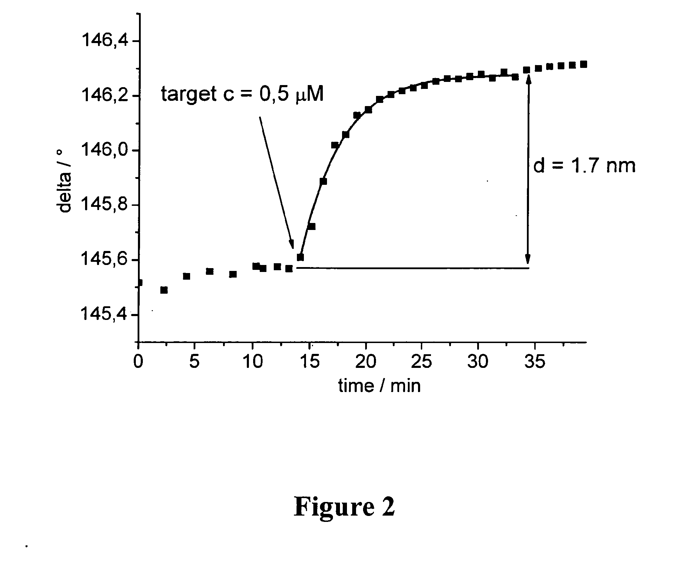

In the following reference will be made to the figure 1 which shows the immobilization of a 30 mer oligonucleotide from solution to a mercaptosilane monolayer on a Si wafer. Figure 2 shows the hybridization of a 30 mer target oligonucleotide from solution to a nanoparticle assisted complementary probe immobilized to a Si wafer.

Reference is now made to the following examples which are given to illustrate, not to limit the invention.

### Examples

### Example 1

### Measurement of immobilization of single stranded oligonucleotide to a substrate surface

The surface of the Si substrate is cleaned with Piranha solution (70% H₂SO₄, 30% H₂O₂, Note: strong oxidizing agent, handle with appropriate precaution) and functionalized with a surface layer supplying the corresponding anchor groups for the probe molecules (2% 3-mercaptopropyltriethoxysilane in Toluene).
The immobilization of the thiolated 30mer (HS-polyT, *c*_{*o*} = 7µM in PBS) was measured by time dependent ellipsometry (60° incoming angle, laser wavelength 633 nm) in a liquid cell. The ellipsometric parameters Delta and Psi were recorded during the immobilization process. The time dependency of Delta was evaluated according to the Langmuir model resulting a association constant *k*_{*on*} for the binding process of *k*_{*on*} = 7.5 ± 0.2 10⁵ M⁻¹s⁻¹. The increase of layer thickness through immobilization (*d* = 1.9 ± 0.1 nm) indicated in figure 1 was determined by an optical model of the layer architecture assuming a refractive index *n* = 1.45 of the organic layers, and a fitting process comparing the theoretical values of the model with the experimental data while adjusting the questionable layer thickness in physically meaningful steps.

### Example 2

### Measurement of hybridization by ellipsometry

The surface of the Si substrate is cleaned with Piranha solution (see above) and functionalized with a surface layer supplying the corresponding anchor groups for the probe molecules (1% 3-aminopropyltriethoxysilane in Toluene). The probe 30mers (HS-polyT) are covalently attached to gold nanoparticles (diameter 12 nm) and immobilized to the substrate in PBS buffer for 2 hours.
The complementary target 30mer polyA is applied to the liquid cell in a concentration *c*_{*o*} = 1 µM in PBS and the ellipsometric parameters Delta and Psi were recorded during the hybridization process.
The association rate constant of the hybridization reaction is determined using the Langmuir model to be *k*_{*on*} = 1.2 ± 0.1 10³ M⁻¹s⁻¹.

The increase of layer thickness through hybridization (*d* = 1.7 ± 0.1 nm) indicated in figure 1 was determined by an optical model of the layer architecture assuming a refractive index *n* = 1.45 of the organic layers, and performing corresponding fitting calculations as stated in Example 1.

The features of the present invention disclosed in the specification, the claims and/or in the accompanying drawings, may, both separately, and in any combination thereof, be material for realising the invention in various forms thereof.

## Claims

1. A method of detecting a hybrid, formed of at least two species, on a substrate, comprising the steps:
a) providing a substrate and a first species,
b) attaching said first species to said substrate,
c) providing a second species, suspected of being able to interact with said first species so as to form a hybrid,
d) allowing formation of said hybrid, if any, out of said first species and said second species to occur, and
e) following said formation of said hybrid over time by means of ellipsometry.

2. The method according to claim 1, wherein said hybrid, formed of at least two species, is a complex of said at least two species.

3. The method according to any of the foregoing claims, wherein said step b) occurs via adsorption or covalent linkage.

4. The method according to any of the foregoing claims, which comprises the optional step:
ba) following the attachment of said first species to said substrate over time by means of ellipsometry.

5. The method according to any of claims 1 - 4, wherein any of steps a) - e) and any substeps occurs without any radioactive, fluorescent, other optical and/or enzymatic label being present.

6. The method according to any of the foregoing claims, wherein said formation of said hybrid is allowed to occur in solution, preferably aqueous solution.

7. The method according to any of the foregoing claims, wherein said formation of said hybrid is allowed to occur under the influence of an electric field, preferably an AC electric field.

8. The method according to any of claims 6-7, wherein said formation of said hybrid is allowed to occur at a pH in the range of from 2 - 12, an ionic strength in the range of from 1 mM to 1 M, and/or a temperature in the range of from 10°C to 80°C.

9. The method according to any of the foregoing claims, wherein said formation of said hybrid is recorded as a signal-over-time-ellipsometry-data so as to allow a subsequent analysis of said signal-over-time-data.

10. The method according to claim 9, wherein said ellipsometry is performed using a data acquisition rate of > 0.2 Hz, an incident wavelength in the range of from 380 nm to 900 nm and an incident angle in the range of from 40° - 80°.

11. The method according to any of the foregoing claims, wherein said ellipsometry is selected from the group comprising ellipsometry and attenuated-total-reflection ellipsometry (ATR).

12. The method according to any of the foregoing claims, wherein said substrate is selected from the group comprising metals, preferably gold, Si, and glass.

13. The method according to any of the foregoing claims, wherein said first species is attached to nanoparticles or nanorods of a material selected from the group comprising metals, preferably gold, silica, carbon and polymeric substances.

14. The method according to claim 13, wherein said first species are attached to said nanoparticles or said nanorods before step b) or step c).

15. The method according to any of the foregoing claims, wherein said at least two species are selected from the group comprising nucleic acids and proteins.

16. The method according to any of claims 1-14, wherein said first species is an antibody and said second species is an antigen or vice versa.

17. The method according to any of claims 1-15, wherein said first species is a nucleic acid or a protein or a protein-nucleic acid complex, and said second species is a nucleic acid or a protein or a protein-nucleic acid-complex.

18. The method according to claim 17, wherein said first species is a nucleic acid and said second species is a nucleic acid.

19. The method according to claim 18, wherein said first species is a single-stranded nucleic acid and said second species is a single-stranded nucleic acid.

20. The method according to claim 19, wherein said second single-stranded nucleic acid is suspected of being complementary to said first single-stranded nucleic acid over 80%, preferably over 90%, more preferably over 95% of the length of first said single-stranded nucleic acid.

21. The method according to claim 19, wherein said second single-stranded nucleic acid is suspected of being complementary to said first single-stranded nucleic acid over the entire length over said first single-stranded nucleic acid.

22. The method according to any of claims 19-21, wherein said first and/or said second single-stranded nucleic acids have a length of 10 - 100, preferably 10-80, more preferably 10 - 50, most preferably 10 - 30 nucleotides.

23. The method according to any of claims 18-22, wherein said first and/or said second nucleic acids are selected from the group comprising DNA, RNA, PNA and other types of modifies nucleic acids.

24. Use of ellipsometry for following the formation of a hybrid, as defined in any of the foregoing claims, from at least two species, as defined in any of the foregoing claims, on a substrate, or the attachment of a species to a substrate, over time.
